# EUROPÄISCHE PATENTANMELDUNG

(11) **EP 3 106 441 A1**
(43) Veröffentlichungstag der Anmeldung: **21.12.2016**
(21) Anmeldenummer: 16169909.5
(22) Anmeldetag: 23.12.2010
(51) Int. Cl.: C03C 10/00, A61C 13/00, C03B 32/02, C04B 41/00, A61C 5/00, A61C 5/08, A61C 5/10, A61C 13/01, A61C 13/083, A61K 6/02, A61K 6/027

(54) **LITHIUMMMETASILIKAT-GLASKERAMIK, VERFAHREN ZU DEREN HERSTELLUNG UND DEREN VERWENDUNG**

(30) Priorität: 23.12.2009 DE 102009060274
(62) Teilanmeldung aus: 10796296.1
(71) Anmelder: Fraunhofer-Gesellschaft zur Förderung der angewandten Forschung e.V., 80686 München (DE); Vita Zahnfabrik H. Rauter GmbH & Co. KG, 79713 Bad Säckingen (DE); DeguDent GmbH, 63457 Hanau (DE)
(72) Erfinder: DURSCHANG, Bernhard, 97228 Rottendorf (DE); PROBST, Jörn, 97273 Kürnach (DE); THIEL, Norbert, 79713 Bad Säckingen (DE); BIBUS, Joachim, 79713 Bad Säckingen (DE); VOLLMANN, Markus, 63571 Gelnhausen (DE); SCHUSSER, Udo, 63755 Alzenau (DE)
(74) Vertreter: Pfenning, Meinig & Partner mbB

(57) **Zusammenfassung**

Die Erfindung betrifft Glaskeramiken auf Basis des Lithiummetasilikat-Systems, die einfach mechanisch bearbeitbar sind und nach der vollständigen Kristallisation eine hochfeste, hoch transluzente und chemisch stabile Glaskeramik darstellen. Ebenso betrifft die Erfindung ein Verfahren zur Herstellung dieser Glaskeramiken. Verwendung finden die erfindungsgemäßen Glaskeramiken als Dentalmaterial.

## Beschreibung

Die Erfindung betrifft Glaskeramiken auf Basis des Lithiumdisilikat-Systems, die in einer Zwischenstufe der Kristallisation einfach mechanisch bearbeitbar sind und nach der vollständigen Kristallisation eine hochfeste, hoch transluzente und chemisch stabile Glaskeramik darstellen. Ebenso betrifft die Erfindung ein Verfahren zur Herstellung dieser Glaskeramiken. Verwendung finden die erfindungsgemäßen Glaskeramiken als Dentalmaterial.

Lithiumdisilikat-Glaskeramiken sind aus der Literatur gut bekannt und mehrere Patente basieren auf diesem Glaskeramiksystem. So werden z.B. in der EP-B-536 479 selbstglasierte Lithiumdisilikat-Glaskeramik-Gegenstände für die Herstellung von Tafelgeschirr beschrieben, in der EP-B-536 572 Lithiumdisilikat-Glaskeramiken, die durch das Aufstreuen eines feinteiligen gefärbten Glases auf ihre Oberfläche als Auskleidungselemente für Bauzwecke eingesetzt werden können.

Ein Schwerpunkt der patentierten Lithiumdisilikat-glaskeramiken liegt in dentalen Anwendungen. Dies liegt daran, dass die Kristallisation der Lithiumdisilikatkristalle über eine Phase geringerer Festigkeit (Lithiummetasilicat) erfolgt und das Materialsystem dadurch u.a. Chairside-Verfahren zugänglich ist (siehe S.D. Stookey: "Chemical Machining of Photosensitive Glass", Ind. Eng. Chem., 45, S. 115-118 (1993) and S.D. Stookey: "Photosensitively Opacifiable Glass" US-A-2,684,911 (1954)). Untersuchung von Borom, z.B. M.-P. Borom, A.M. Turkalo, R.H. Doremus: "Strength and Microstructure in Lithium Disilicate Glass-Ceramics", J. Am. Ceream. Soc., 58, Nr. 9-10, S. 385-391 (1975) und M.-P. Borom, A.M. Turkalo, R.H. Doremus: "Verfahren zum Herstellen von Glaskeramiken" DE-A-24 51 121 (1974) zeigen, dass Glaskeramiken, die Lithiummetasilikat als Hauptphase enthalten, eine verringerte Festigkeit im Vergleich zu Glaskeramiken haben, die Lithiumdisilikat als einzige kristalline Phase enthalten.

Dieses Prinzip wurde ausgenutzt, um in einem zweistufigen Kristallisationsprozess zuerst eine Glaskeramik herzustellen, die mechanisch gut bearbeitbar ist, z.B. mittels CAD/CAM-Verfahren, und diese anschließend in einer zweiten Kristallisationsstufe zur Dentalglaskeramik zu prozessieren. Dieses Verfahren ist geeignet, um dentale Restaurationen nach dem sog. chair-side-Verfahren verwenden zu können. Bei diesem Verfahren wird in der Zahnarztpraxis aus einem Glaskeramikblock nach der ersten Kristallisationsstufe mittels CAD/CAM eine individuell angepasste Krone/Onlay/Inlay herausgefräst, diese in einem Spezialofen der zweiten Kristallisationsstufe unterzogen und direkt in der ersten und einzigen Zahnarztsitzung dem Patienten eingesetzt (DE 10 2005 028 637).

Außerdem wurden in WO-A-95/32678 und US-A-5,507,981 Lithiumdisilikat-Glaskeramiken beschrieben, die durch den Einsatz eines speziellen verpressbaren Tiegels durch Heißpressen zu geformten Dentalprodukten verarbeitet werden können. Weiter sind aus der DE-C-14 21 886 Glaskeramiken auf Basis von SiO₂ und Li₂O bekannt, welche große Mengen an physiologisch sehr bedenklichem Arsenoxid enthalten. Auch in US-A-4,515,634 und in FR-A-2 655 264 werden zur Herstellung von Dentalkronen und -brücken geeignete Lithiumdisilikat-Glaskeramiken offenbart.

Alle bekannten Lithiumdisilikat-Glaskeramiken zeigen Unzulänglichkeiten bei ihrer Verarbeitung zu geformten Produkten und/oder mechanischen bzw. optischen Eigenschaften und/oder chemischen Stabilität. Insbesondere sind beim Einsatz im Dentalbereich für alle genannten Eigenschaften gleichermaßen hohe Ansprüche zu erfüllen.

Ausgehend hiervon war es Aufgabe der vorliegenden Erfindung, eine Glaskeramik bereitzustellen, die verbesserte mechanische und optische Eigenschaften sowie eine verbesserte chemische Stabilität gegenüber den aus dem Stand der Technik bekannten (Glas-)Keramiken aufweist.

Diese Aufgabe wird durch die Lithiumdisilikat-Glaskeramik mit den Merkmalen des Anspruchs 1, sowie durch das Verfahren zur Herstellung dieser Glaskeramik mit den Merkmalen des Anspruchs 9 gelöst. In Anspruch 12 werden erfindungsgemäße Verwendungen angegeben. Ebenso wird ein geformtes Dentalprodukt mit den Merkmalen des Anspruchs 13 bereitgestellt. Die weiteren abhängigen Ansprüche zeigen vorteilhafte Weiterbildungen auf.

Im Rahmen der vorliegenden Erfindung wurden Glaszusammensetzungen entwickelt, die sich in einem zweistufigen Herstellungsprozess darstellen lassen, nach der ersten Kristallisationsstufe, insbesondere mittels CAD/CAM gut zu bearbeiten sind und nach einer sehr kurzen zweiten Kristallisationsstufe sowohl hochtransparent und hochfest sind als auch bessere chemische Beständigkeiten aufweisen, als die bekannten Lithiumdisilikat-Glaskeramiken.

Überraschenderweise hat sich gezeigt, dass die Zugabe von ZrO₂ zu bestimmten Glaszusammensetzungen zu Glaskeramiken führt, die in einer Kristallisationszwischenstufe sehr gut bearbeitbar sind und im Endzustand hervorragende Festigkeitswerte, außerordentliche Transluzenz und deutlich erhöhte chemische Beständigkeiten aufweisen.

Es zeigte sich, dass sich bis zu 20 Gew.-% eines Stabilisators ausgewählt aus der Gruppe bestehend aus ZrO₂, HfO₂ oder Mischungen hiervon, in das Glas einbauen lässt, ohne die Struktur signifikant zu beeinflussen. Entgegen allen Erwartungen kristallisiert der Stabilisator hierbei nicht als eigene Kristallphase aus, sondern verbleibt in der Restglasphase. Durch den hohen Anteil in der amorphen Phase werden in dieser Phase die mechanischen und chemischen Beständigkeiten enorm verbessert, was auch zu verbesserten Eigenschaften im Endprodukt führt. Insbesondere die chemische Beständigkeit lässt sich über die Zusammensetzung der Restglasphase verbessern, da die Glasphase eine deutliche höhere Löslichkeit aufweist als das Lithiumdisilikat und somit die Schwachstelle bezüglich chemischem Angriff darstellt. Die extrem hohe Löslichkeit des Stabilisators (ZrO₂) in der Glasphase ist insbesondere beachtenswert, da z.B. Zirkonoxid bei vielen silikatischen Glaskeramiken als Keimbildner fungiert, d.h. bei einer Temperaturbehandlung als erste Phase auskristallisiert und an diesen ZrO₂-Kristallen sich die eigentlich angestrebte Kristallphase erleichtert und feinkristallin ausscheidet.

Durch die hohen Anteile des Stabilisators, die im Wesentlichen in der amorphen Phase verbleiben, ist der kristalline Anteil entsprechend begrenzt. Hierdurch und durch die geringe Kristallitgröße der Lithiumdisilikatkristalle entsteht eine gute Transluzenz der Materialien nach der zweiten Kristallisation. Die Transluzenz wird jedoch auch noch dadurch verbessert, dass der Brechungsindex der Glasphase wiederum durch den Stabilisator erhöht wird und sich dadurch dem Brechungsindex des Lithiumdisilikats anpasst. Bei Glaskeramiken, bei denen der Brechungsindex der amorphen Matrixphase mit dem Brechungsindex der kristallinen Phase/Phasen übereinstimmt, findet man sehr gute Transluzenzeigenschaften, relativ unabhängig von der Kristallitgröße. In den erfindungsgemäßen Glaskeramiken sind also alle drei Punkte zur Erzeugung einer höchst transluzenten Glaskeramik erfüllt:
- limitierter Kristallphasenanteil,
- kleine Kristalle (< 500 nm),
- angepasster Brechungsindex von amorpher und kristalliner Phase.

Der hohe Anteil des Stabilisators wirkt sich in der Glaskeramik also in
- einer verbesserten chemischen Beständigkeit,
- höheren Festigkeitswerten und
- einer in mehrerer Hinsicht verbesserten Transluzenz
zu entsprechenden Glaskeramiken ohne bzw. mit geringem ZrO₂- oder HfO₂-Anteil aus.

Die erfindungsgemäßen Glaskeramiken können vorzugsweise mittels eines Verfahrens hergestellt werden, bei dem
(a) ein Ausgangsglas hergestellt wird, das die Komponenten der Glaskeramik enthält,
(b) das Ausgangsglas einer ersten Wärmebehandlung bei einer ersten Temperatur unterworfen wird, um eine Glaskeramik herzustellen, welche Lithiummetasilikat als einzige bzw. Hauptkristallphase aufweist und
(c) diese Glaskeramik einer zweiten Wärmebehandlung unterzogen wird, bei der das Lithiummetasilikat mit SiO₂ aus der Glasphase in Lithiumdisilikat umgewandelt wird und anschließend Lithiumdisilikat als einzige oder Hauptkristallphase vorliegt.

Die Kristallisation zum Lithiummetasilikat findet vorzugsweise bei Temperaturen zwischen 620 °C und 800 °C statt, mit Zeiten zwischen 1 und 200 Minuten, bevorzugt zwischen 650 °C und 750 °C für 10 bis 60 Minuten.

Die Kristallisation zum Lithiumdisilikat findet vorzugsweise bei Temperaturen zwischen 800 °C und 1040 °C statt, mit Zeiten von 5 bis 200 Minuten, bevorzugt zwischen 800 °C und 870 °C für 5 bis 30 Minuten.

Anhand der nachfolgenden Beispiele soll der erfindungsgemäße Gegenstand näher erläutert werden, ohne diesen auf die hier gezeigten speziellen Ausführungsformen einschränken zu wollen.

### Beispiele 1 bis 6

In den Beispielen 1 bis 6 sind Zusammensetzungen von hochzirkonoxidhaltigen Gläsern angegeben, die durch eine zweistufige Temperaturbehandlung erst zu gut mechanisch bearbeitbaren Lithiummetasilicat-Glaskeramiken und anschließend zu hoch-transluzenten, hoch-festen und chemisch stabilen Lithiumdisilicat-Glaskeramiken überführt werden.

Die Zusammensetzungen mit ihren Bestandteilen sind in Tabelle 1 dargestellt.

**Tabelle 1**

| | **B1** | **B2** | **B3** | **B4** | **B5** | **B6** |
|---|---|---|---|---|---|---|
| SiO₂ | 66, 9 | 65, 8 | 65,5 | 63,7 | 63,5 | 63,5 |
| Li₂O | 13, 9 | 13,7 | 13, 6 | 13,2 | 14,4 | 12, 9 |
| ZrO₂ | 10,0 | 10,0 | 12, 0 | 11,7 | 12,7 | 13,5 |
| Al₂O₃ | 3,2 | 3,1 | 3,1 | 3, 0 | 3,3 | 3,5 |
| P₂O₅ | 3, 0 | 3, 0 | 3, 0 | 2, 9 | 3,1 | 3,4 |
| K₂O | 2, 9 | 2, 9 | 2, 9 | 2,8 | 3, 0 | 3,2 |
| CeO₂ | - | 1,0 | - | 2,0 | - | - |
| Er₂O₃ | - | 0,2 | - | 0,3 | - | - |
| Tb₂O₃ | - | 0,3 | - | 0,3 | - | - |

Die Gläser wurden bei 1500 °C erschmolzen und in Metallformen zu Blocks gegossen. Die Blocks wurden bei 560 °C im Ofen entspannt und langsam abgekühlt. Für die unterschiedlichen Charakterisierungsverfahren wurden die Glasblocks zerteilt und einer ersten Kristallisationsbehandlung unterzogen. Hierfür wurden die Gläser für 10 bis 120 Minuten bei 600 °C bis 750 °C ausgelagert. Hierdurch wurden Glaskeramiken mit Festigkeitswerten von 150 MPa bis 220 MPa hergestellt. Als Kristallphase wurde hierbei ausschließlich Lithiummetasilicat festgestellt. In diesem Zustand ist eine Bearbeitung mittels CAD/CAM-Methoden sehr gut möglich.

Mit einer zweiten kurzen Kristallisation bei 800 °C bis 950 °C für 3 bis 15 Minuten findet eine Umkristallisation des Lithiummetasilikates mit amorphem SiO₂ aus der Glasphase zum Lithiumdisilikat statt und es kommt zu einer Festigkeitserhöhung auf 300 MPa bis 450 MPa. Neben der Lithiumdisilikatphase kann hierbei eine zirkonoxidhaltige Nebenkristallphase entstehen. Außerdem können noch geringe Reste von Lithiummetasilikat vorliegen. Die eindeutige Hauptkristallphase ist das Lithiumdisilikat.

In Tabelle 2 sind die Kristallisationsbedingungen von einzelnen Gläsern sowie die entstehenden Kristallphasen und Festigkeitswerte dargestellt.

**Tabelle 2**

| **Glas** | **B1** | **B2** | **B3** | **B4** | **B5** | **B6** |
|---|---|---|---|---|---|---|
| 1. Kristallisation | 650 °C | 700 °C | 650 °C | 700 °C | 700 °C | 700 °C |
| | 20 min | 40 min | 30 min | 20 min | 40 min | 40 min |
| 2. Kristallisation | 850 °C | 830 °C | 870 °C | 850 °C | 820 °C | 830 °C |
| | 10 min | 10 min | 20 min | 8 min | 10 min | 10 min |
| Kristallphasen | | | | | | |
| - Hauptphase (> 80 %) | Disilikat | Disilikat | Disilikat | Disilikat | Disilikat | Disilikat |
| - Nebenphase (< 20 %) | - | - | - | - | Metasilikat | Metasilikat |
| Transluzenz | hervorragend | sehr gut | hervorragend | sehr gut | hervorragend | hervorragend |
| 3-Punkt-Biegebr.-Festigkeit | 375 MPa | 413 MPa | 380 MPa | 418 MPa | 356 MPa | 385 MPa |

Die Erfindung betrifft weiterhin die folgenden Aspekte:
1. Lithiumdisilikat-Glaskeramik enthaltend mindestens 10 Gew.-% eines Stabilisators zur Erhöhung der chemischen und mechanischen Stabilität, wobei der Stabilisator im Wesentlichen in der amorphen Phase vorliegt.
2. Lithiumdisilikat-Glaskeramik nach Aspekt 1, dadurch gekennzeichnet, dass der Stabilisator ausgewählt ist aus der Gruppe bestehend aus Zirkonoxid, Hafniumoxid und Mischungen hiervon.
3. Lithiumdisilikat-Glaskeramik nach Aspekt 1 oder 2 mit folgender Zusammensetzung: 55 bis 70 Gew.-% SiO₂,
   10 bis 15 Gew.-% LiO₂,
   10 bis 20 Gew.-% des Stabilisators ausgewählt aus der Gruppe bestehend aus ZrO₂, HfO₂ oder Mischungen hiervon,
   0,1 bis 5 Gew.-% K₂O,
   0,1 bis 5 Gew.-% Al₂O₃,
   0 bis 10 Gew.-% an Zusatzstoffen sowie
   0 bis 10 Gew.-% an Farbstoffen.
4. Lithiumdisilikat-Glaskeramik nach einem der vorhergehenden Aspekte,
   dadurch gekennzeichnet, dass die Farbstoffe glasfärbende Oxide und/oder Farbkörper sind.
5. Lithiumdisilikat-Glaskeramik nach einem der vorhergehenden Aspekte,
   dadurch gekennzeichnet, dass die glasfärbenden Oxide ausgewählt sind aus der Gruppe der Oxide von Eisen, Titan, Cer, Kupfer, Chrom, Kobalt, Nickel, Mangan, Selen, Silber, Indium, Gold Seltenerdmetallen, insbesondere Neodym, Praesodym, Samarium und Europium.
6. Lithiumdisilikat-Glaskeramik nach einem der vorhergehenden Aspekte,
   dadurch gekennzeichnet, dass die Farbkörper dotierte Spinelle sind.
7. Lithiumdisilikat-Glaskeramik nach einem der vorhergehenden Aspekte,
   dadurch gekennzeichnet, dass die Zusatzstoffe ausgewählt sind aus der Gruppe bestehend aus Boroxid, Phosphoroxid, Fluor, Natriumoxid, Bariumoxid, Strontiumoxid, Magnesiumoxid, Zinkoxid, Calciumoxid, Yttriumoxid, Titanoxid, Nioboxid, Tantaloxid, Lanthanoxid und Mischungen hiervon.
8. Lithiumdisilikat-Glaskeramik nach einem der vorhergehenden Aspekte mit folgender Zusammensetzung:
   58 bis 64 Gew.-% SiO₂,
   11 bis 13 Gew.-% LiO₂,
   10 bis 15 Gew.-% des Stabilisators ausgewählt aus der Gruppe bestehend aus ZrO₂, HfO₂ oder Mischungen hiervon,
   2 bis 5 Gew.-% K₂O,
   2 bis 5 Gew.-% Al₂O₃,
   2 bis 5 Gew.-% P₂O₅ sowie
   0 bis 5 Gew.-% an Zusatzstoffen sowie
   0 bis 10 Gew.-% an Farbstoffen.
9. Verfahren zur Herstellung einer Lithiumdisilikat-Glaskeramik nach einem der vorhergehenden Aspekte, bei dem
   a) ein Ausgangsglas hergestellt wird, das die Komponenten der Glaskeramik enthält,
   b) das Ausgangsglas einer ersten Wärmebehandlung zur Herstellung einer Glaskeramik, die Lithiummetasilicat als Hauptkristallphase aufweist, unterworfen wird,
   c) die Glaskeramik aus b) einer zweiten Wärmebehandlung unterworfen wird, bei der das Lithiummetasilicat mit SiO₂ aus der Glasphase in Lithiumdisilicat umgewandelt wird und anschließend Lithiumdisilicat als Hauptkristallphase vorliegt.
10. Verfahren nach Aspekt 9,
   dadurch gekennzeichnet, dass die erste Wärmebehandlung bei einer Temperatur von 620 °C bis 800 °C über einen Zeitraum von 1 bis 200 min, insbesondere von 650 °C bis 750 °C über einen Zeitraum von 10 bis 60 min, erfolgt.
11. Verfahren nach einem der Aspekte 9 oder 10,
   dadurch gekennzeichnet, dass die zweite Wärmebehandlung bei einer Temperatur von 800 °C bis 1040 °C über einen Zeitraum von 5 bis 200 min, insbesondere von 650 °C bis 750 °C über einen Zeitraum von 5 bis 30 min erfolgt.
12. Verwendung der Lithiumdisilikat-Glaskeramik nach einem der Aspekte 1 bis 8 als Dentalmaterial oder als Komponente eines Dentalmaterials.
13. Geformtes Dentalprodukt enthaltend eine Lithiumdisilikat-Glaskeramik nach einem der Aspekte 1 bis 8, insbesondere in Form eines Inlays, eines Onlays, einer Brücke, eines Stiftaufbaus, einer Verblendung, einer (Teil)krone.

## Patentansprüche

1. Glaskeramik, die Lithiummetasilicat als Hauptkristallphase aufweist, mit folgender Zusammensetzung:
55 bis 70 Gew.-% SiO₂,
10 bis 15 Gew.-% Li₂O,
10 bis 20 Gew.-% eines Stabilisators ausgewählt aus der Gruppe bestehend aus ZrO₂, HfO₂ oder Mischungen hiervon,
0,1 bis 5 Gew.-% K₂O,
0,1 bis 5 Gew.-% Al₂O₃,
0 bis 10 Gew.-% an Zusatzstoffen sowie
0 bis 10 Gew.-% an Farbstoffen,
wobei der Stabilisator im Wesentlichen in der amorphen Phase vorliegt.

2. Glaskeramik nach Anspruch 1,
**dadurch gekennzeichnet, dass** die Farbstoffe glasfärbende Oxide und/oder Farbkörper sind.

3. Glaskeramik nach einem der vorhergehenden Ansprüche,
**dadurch gekennzeichnet, dass** die glasfärbenden Oxide ausgewählt sind aus der Gruppe der Oxide von Eisen, Titan, Cer, Kupfer, Chrom, Kobalt, Nickel, Mangan, Selen, Silber, Indium, Gold Seltenerdmetallen, insbesondere Neodym, Praseodym, Samarium und Europium.

4. Glaskeramik nach einem der vorhergehenden Ansprüche,
**dadurch gekennzeichnet, dass** die Farbkörper dotierte Spinelle sind.

5. Glaskeramik nach einem der vorhergehenden Ansprüche,
**dadurch gekennzeichnet, dass** die Zusatzstoffe ausgewählt sind aus der Gruppe bestehend aus
Boroxid, Phosphoroxid, Fluor, Natriumoxid, Bariumoxid, Strontiumoxid, Magnesiumoxid, Zinkoxid, Calciumoxid, Yttriumoxid, Titanoxid, Nioboxid, Tantaloxid, Lanthanoxid und Mischungen hiervon.

6. Glaskeramik nach einem der vorhergehenden Ansprüche mit folgender Zusammensetzung:
58 bis 64 Gew.-% SiO₂,
11 bis 13 Gew.-% Li₂O,
10 bis 15 Gew.-% des Stabilisators ausgewählt aus der Gruppe bestehend aus ZrO₂, HfO₂ oder Mischungen hiervon,
2 bis 5 Gew.-% K₂O,
2 bis 5 Gew.-% Al₂O₃,
2 bis 5 Gew.-% P₂O₅ sowie
0 bis 5 Gew.-% an Zusatzstoffen sowie
0 bis 10 Gew.-% an Farbstoffen.

7. Verfahren zur Herstellung einer Lithiummetasilikat-Glaskeramik nach einem der vorhergehenden Ansprüche, bei dem
a) ein Ausgangsglas hergestellt wird, das die Komponenten der Glaskeramik enthält,
b) das Ausgangsglas einer Wärmebehandlung zur Herstellung einer Glaskeramik, die Lithiummetasilicat als Hauptkristallphase aufweist, unterworfen wird.

8. Verfahren nach Anspruch 7,
**dadurch gekennzeichnet, dass** die Wärmebehandlung bei einer Temperatur von 620 °C bis 800 °C über einen Zeitraum von 1 bis 200 min, insbesondere von 650 °C bis 750 °C über einen Zeitraum von 10 bis 60 min, erfolgt.
